Europäisches Patentamt

European Patent Office

Office européen des brevets

(11) Publication number: **0 377 540**
**A1**

(12) **EUROPEAN PATENT APPLICATION**

(21) Application number: **90400043.7**

(22) Date of filing: **05.01.90**

(51) Int. Cl.5: **C12N 15/18, C12P 21/02, C07K 13/00, C12N 1/21, A23K 1/165, A61K 37/36, //(C12N1/21,C12R1:19)**

(30) Priority: **06.01.89 EP 89400047**

(43) Date of publication of application: **11.07.90 Bulletin 90/28**

(84) Designated Contracting States: **BE DE ES FR GR IT**

(71) Applicant: **EUROGENTEC S.A.**
**Campus du Sart-Tilman Allée du Six Août B6 Bldg**
**B-4000 Liège(BE)**

(72) Inventor: **The designation of the inventor has not yet been filed**

(74) Representative: **Grosset-Fournier, Chantal Catherine et al**
**SC Ernest Gutmann/Yves Plasseraud 67 boulevard Haussmann**
**F-75008 Paris(FR)**

(54) **Recombinant fish hormone proteins.**

(57) The present invention relates to a recombinant stable polypeptide comprising in its polypeptide chain an aminoacid sequence contained in one of the following amino sequences :

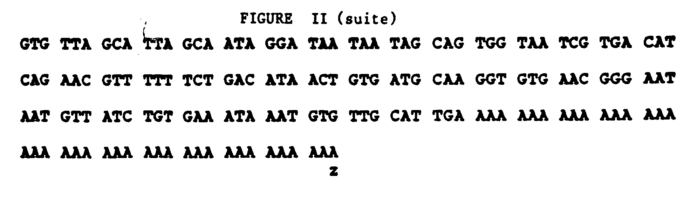

* from amino acid (-20) to amino acid (187) represented in Fig. 2,
* from amino acid (1) to amino acid (187) represented in Fig. 2,
* from amino acid (1) to amino acid (187) represented in Fig. 2, preceded by Met.

FIGURE II (suite)

```
GTG TTA GCA TTA GCA ATA GGA TAA TAA TAG CAG TGG TAA TCG TGA CAT
CAG AAC GTT TTT TCT GAC ATA ACT GTG ATG CAA GGT GTG AAC GGG AAT
AAT GTT ATC TGT GAA ATA AAT GTG TTG CAT TGA AAA AAA AAA AAA AAA
AAA AAA AAA AAA AAA AAA AAA AAA
                            2
```

FIGURE II

```
                                    -20
                          Met Pro Ala Met Asn Ser Val
   CT CGC CCG CAA ACA GAG CCT GAA CTG ATG CCA GCC ATG AAC TCA GTC
-26                                  1
        -10                                      -1  1
Val Leu Leu Leu Ser Val Val Cys Leu Gly Val Ser Ser Gln Gln Ile
GTC CTC CTC CTG TCG GTT GTG TGT TTG GGC GTC TCC TCT CAG CAG ATC
                                                      61
         10
Thr Asp Ser Gln Arg Leu Phe Ser Ile Ala Val Asn Arg Val Thr His
ACA GAC AGC CAG CGT TTG TTC TCC ATT GCA GTC AAC AGA GTC ACG CAC
    20                      30
Leu His Leu Leu Ala Gln Arg Leu Phe Ser Asp Phe Glu Ser Ser Leu
CTG CAC CTG CTC GCC CAG ACA CTC TTC TCG GAC TTT GAG AGC TCT CTG
             40                              50
Gln Thr Glu Glu Gln Arg Gln Leu Asn Lys Ile Phe Leu Gln Asp Phe
CAG ACG GAG GAG CAA CGT CAG CTC AAC AAA ATC TTC CTG CAG GAC TTC
         60
Cys Asn Ser Asp Tyr Ile Ile Ser Pro Ile Asp Lys His Glu Thr Gln
TGC AAC TCT GAT TAC ATC ATC AGC CCG ATC GAC AAA CAC GAG ACG CAG
       70                          80
Arg Ser Ser Val Leu Lys Leu Leu Ser Ile Ser Tyr Gly Leu Val Glu
CGC AGC TCG GTC CTG AAG CTC CTG TCG ATC TCC TAT GGA CTG GTT GAG
                             90
Ser Trp Glu Phe Pro Ser Arg Ser Leu Ser Gly Ser Ser Leu Arg
TCC TGG GAG TTT CCC AGT CGC TCT CTG TCT GGT TCC TCT CTG ACG
100                         110
Asn Gln Ile Ser Pro Arg Leu Ser Glu Leu Lys Thr Gly Ile Leu Leu
AAC CAG ATT TCA CCA AGG CTC TCT GAG CTT AAA ACG GGA ATC TTG CTG
         120                              130
Leu Ile Arg Ala Asn Gln Asp Glu Ala Glu Asn Tyr Pro Asp Thr Asp
CTC ATC AGG GCC AAT CAG GAT GAA GCA GAG AAT TAT CCT GAC ACC GAC
                       140
Thr Leu Gln His Ala Pro Tyr Gly Asn Tyr Tyr Gln Ser Leu Gly Gly
ACC CTC CAG CAC GCT CCT TAC GGA AAC TAT TAT CAA AGT CTG GGA GGC
     150                              160
Asn Glu Ser Leu Arg Gln Thr Tyr Glu Leu Leu Ala Cys Phe Lys Lys
AAC GAA TCG CTG AGA CAA ACT TAT GAA TTG CTG GCT TGC TTC AAG AAG
                   170
Asp Met His Lys Val Glu Thr Tyr Leu Thr Val Ala Lys Cys Arg Leu
GAC ATG CAC AAG GTG GAG ACC TAC CTC ACG GTA GCT AAA TGT CGA CTC
180                     187
Ser Pro Glu Ala Asn Cys Thr Leu
TCT CCA GAA GCA AAC TGC ACT CTG TAG CTC CAC CTA ATA TTG ATA CTG
                           621
ATA CGT GCT CTG TAG CCC CAC CCT CAT GTT GGC AAA CTC TGC TTA CAT
```

## RECOMBINANT FISH HORMONE PROTEINS

The present invention relates to recombinant fish growth hormones exhibiting growth stimulation and osmoregulatory effects. It also relates to a process for producing large quantities of growth hormones in microorganisms harbouring genes cloned from warm water fish species, coding for said growth hormones.

Growth hormones (hereafter denoted GH) constitute a family of evolutionary proteins produced by the pituitary gland in all vertebrates. In general, these pituitary hormone proteins display pleiotrophic biological activities. In fish these activities range from growth stimulation and osmoregulatory adaptation to water salinity. Most studies of these hormones have focussed on their growth promoting and osmoregulatory activities, demonstrating their role in seawater adaptation, and suggesting their potential application in the field of fish culture. Recently, several growth hormones have been isolated and cloned from various cold water fish species such as salmon, trout, carp and tuna (Sebine et al. 1985 ; Agellon and Chen 1986 ; Nicoll et al. 1988 ; Gonzalez-Villasenor 1988 ; Sato et al. 1988 ; Yasuda et al. 1987 ; Song et al. 1988). Attemps to produce recombinant growth hormones by expressing genes cloned from these fish in microorganisms have thus far met with little success.

In the present invention cloning techniques have been used to isolate the genes coding for the different growth promoting and osmoregulatory pituitary hormones of the warm water fish species such as tilapia, catfish or yellowtail. The warm water fish tilapia is a euryhaline fish which can reproduce in sea water as well as in fresh water. Because of recent development of tilapia aquaculture in brackish or in seawater, interest has focussed on growth promotion and salinity tolerance and hence on the physiology of osmoregulation in this species. Little is known about the tilapia GH.

The invention relates to new proteins or polypeptides, which present a stability with respect to heat and can be used in warm water fish species.

By way of example the tilapia species Oreochromis niloticus has been used and it is shown that one hormone exhibiting growth promoting and/or osmoregulatory activities can be isolated, namely one growth hormone protein (hereafter denoted TGH).

The invention also relates to the nucleic acids, described hereafter, encoding the tilapia or catfish growth hormones and the regulatory signals preceding the coding sequences which allow the coding sequences to be expressed in the host organism including promoter signals which control the transcription of said coding sequences and the translation initiation and termination signals.

The invention also relates to the recombinant DNAs, plasmids, cosmids and phages containing nucleic acid sequences encoding the tilapia or catfish growth hormones and the genetic elements essential for their replication.

In a particular application of the invention, the recombinant DNAs, described hereafter, are plasmid expression vectors containing a functional origin of replication, regulatory elements allowing the expression, in the host organism, of the sequences coding for the tilapia or catfish growth hormones, a promoter which is active in said host organism, and in particular an inducible promoter.

The invention also relates to the host organisms transformed with the recombinant DNAs, described hereabove, capable of replicating in said host organism, and containing regulatory elements allowing the expression of the tilapia or catfish growth hormones in this host organism.

A preferred host organism is Escherichia coli, transformed with recombinant DNAs as described in the examples below.

The present invention also relates to a process for producing recombinant fish growth hormones in microorganisms using the genes encoding for said hormone proteins isolated from warm water fish species particularly tilapia or catfish.

The present invention relates to the culturing of an appropriate host organism previously transformed with a DNA containing nucleotide sequences encoding the tilapia or catfish growth hormones which are under the control of regulatory elements such as a promoter recognized by the RNA polymerase of the host organism, a translation initiation and termination signal, and the purification of the protein products synthesized by the host organism.

The invention also relates in particular to the cultures of host organisms transformed as described below and which are capable of producing the tilapia or catfish growth hormones according to the invention.

The present invention also relates to the products based on the tilapia growth hormone proteins in the industrial culture of fish to enhance the growth of the fish in culture and to improve their adaptation to culturing conditions.

The present invention also relates to preparations based on tilapia growth hormone proteins or mixtures thereof which can be administered to fish cultures using methods such as injection, balneation or spraying.

The present invention also relates to food for fish, containing, besides the classical ingredients, the recombinant polypeptides or proteins produced according to the process of the invention, in an efficient amount.

The invention relates to the following stable recombinant polypeptides : those comprising in their polypeptide chain an aminoacid sequence contained in one of the following amino sequences :

* from amino acid (-20) to amino acid (187) represented in Fig. 2,
* from amino acid (1) to amino acid (187) represented in Fig. 2,
* from amino acid (1) to amino acid (187) represented in Fig. 2, preceded by Met.

The expression "stable polypeptide" means that the polypeptide obtained is stable, even after isolation from the medium culture in which it is produced. More precisely, it means that the polypeptide is stable at the temperature of 28° C to 42° C under which it is synthesized in the cellular host, and is not subject to any rapid degradation, and hence can be expressed in large amounts comprising a substantial fraction of the total cellular protein of said cellular host, substantial fraction meaning for example at least 10% to 80% of the total cellular protein in bacterial host, or for example at least 3% to 50% of the total cellular protein in yeast cellular hosts.

The expression "recombinant polypeptide" is not limited to mere aminoacid sequences, but also encompasses the glycosylated polypeptides or the polypeptides comprising disulfide bridges. The glycosylated polypeptides can be obtained directly by expressing a nucleic acid above and hereafter defined in an appropriate cellular host, for example yeast, or can be obtained by in vitro glycosylation of a non glycosylated recombinant polypeptide.

More particularly, the invention relates to the following polypeptides :
- those containing in their polypeptide chain the following sequences:
* from amino acid (-20) to amino acid (187) represented in Fig. 2,
* from amino acid (1) to amino acid (187) represented in Fig. 2,
* from amino acid (1) to amino acid (187) represented in Fig. 2, preceded by Met,
- or constituted by one of the abovesaid amino acid sequences.

The invention also relates to the signal peptides constituted by the following aminoacid sequence:
- from aminoacid (-20) to aminoacid (-1) of fig. 2.

The invention relates to any recombinant polypeptide constituted by anyone of the above mentioned signal peptides, combined with any other hormones such as growth hormones of fish.

The process for preparing the polypeptides of the invention comprises producing recombinant polypeptides or proteins in typical microorganisms such as Escherichia coli and yeast at a temperature from about 28° C to about 42° C, depending upon the selected microorganism, as a means of obtaining large quantities of stable polypeptides or proteins encoded by cloned genes.

More precisely, the process for preparing a stable recombinant growth hormone of the invention comprises the following steps :
- a cellular host, which is previously transformed by an appropriate vector containing a nucleic acid coding for polypeptides having
. the polypeptide sequence of the natural growth hormone of tilapia or catfish or
. a polypeptide sequence containing part or the totality of the sequence of the natural growth hormones of tilapia or catfish, in so far as the polypeptide sequence produced has growth promoting and/or osmoregulatory properties,
is cultivated in an appropriate medium at a temperature from about 28° C to about 42° C depending on the selected microorganism and
- the polypeptide produced by said transformed cellular host is recovered from the above said medium culture and, if need be, purified.

The polypeptides comprising the disulfide bridges can be obtained either directly by expressing a nucleic acid, defined above and hereafter, in an appropriate cellular host, for example yeast, or indirectly by denaturation and renaturation of purified fractions of the recombinant polypeptide in buffers containing an appropriate standard redox potential.

The temperature used is chosen such as it is compatible with the conditions required to allow the synthesis of proteins in the cellular host. The upper limit of the temperature range is critical because above that temperature all macromolecular synthesis in the cellular host ceases. The lower limit of the temperature range is critical because under that temperature all enzymatic reactions, including macromolecular synthesis, and protein synthesis, are slowed down to such an extend that the polypeptide will not be produced in the large amounts defined hereabove.

In an advantageous process of the invention, the nucleic acids coding for the natural growth hormones are under the control of regulatory elements, such as a promoter recognized by the RNA polymerase of the host organism, a translation initiation and termination signal.

In the process of the invention, the cellular host can advantageously be E. coli, the growth of which is stopped at the exponential phase and the polypeptide produced by said transformed cellular host is recovered from the culture of cells from other proteins produced by E. coli.

E. coli is advantageously used for the production of very large amounts, as the experimental conditions required are well defined.

The cellular host can also be yeast, such as Sacharomycces strains. Yeast is advantageously used for the production of the polypeptide in the culture medium as the polypeptide will be secreted in the culture medium when the gene encoding the polypeptide contains a signal peptide.

Furthermore, yeast is also advantageously used for the production of the biologically active polypeptide since in his host naturally occurring disulfide bridges are formed in newly synthesized proteins.

In the process of the invention, the recombinant polypeptide can be preceded by a signal peptide, particularly by a signal peptide having the same amino acid sequence as the natural signal peptide in tilapia or catfish.

In the process of the invention, the recombinant polypeptide is advantageously encoded by a gene in which the coding sequence of the polypeptides is preceded by an initiator methionine codon.

The latter is required for allowing the translation of the polypeptide to be initiated at the correct codon. The preceding methionine might or not be cleaved off from the polypeptide during its synthesis.

The polypeptides of the invention can not be prepared only by the process of the invention, but can also be prepared by any preparation process, such as classical chemical synthesis.

Another method for preparing the polypeptides of the invention is characterized in that, preferably starting from the C-terminal amino acid, the successive aminoacyls in the requisite order, or aminoacyls and fragments formed beforehand and already containing several aminoacyl residues in the appropriate order, or alternatively several fragments prepared in this manner beforehand, are condensed successively in pairs, it being understood that care will have been taken to protect beforehand all the reactive groups carried by these aminoacyls or fragments except for the amine groups of one and carboxyl group of the other, or vice versa, which must normally participate in peptide bond formation, in particular after activation of the carboxyl group, according to methods known in peptide synthesis, and so on, proceeding stepwise up to the N-terminal amino acid.

The invention also relates to the nucleic acids comprising a nucleotide chain coding for the following aminoacid sequence; - those contained in one of the following sequences :

* from amino acid (-20) to amino acid (187) represented in Fig. 2,
* from amino acid (1) to amino acid (187) represented in Fig. 2,
* from amino acid (1) to amino acid (187) represented in Fig. 2, preceded by Met,
- or those comprising one of the abovesaid amino acid sequence or constituted by one of the abovesaid amino acid sequences.

Advantageous nucleic acids of the invention can also be defined as those comprising a part or the totality of one of the following nucleotidic sequences:

* from nucleotide (-26) to nucleotide (Z) of Fig. 2,
* from nucleotide (1) to nucleotide (621) of figure 2,
* from nucleotide (61) to nucleotide (621) of Fig. 2,
* from nucleotide (61) to nucleotide (621) of Fig. 2, preceded by the AUG codon,

or constituted by one of the above mentioned nucleotide sequences.

The invention also relates to the nucleic acids coding for the above defined signal peptide.

The invention also relates to recombinant vectors, particularly for the cloning and/or expression, particularly of the plasmid, cosmid or phage type, containing a nucleic acid coding for a natural polypeptide - presenting growth promoting and/or osmoregulatory properties - of tilapia or catfish in particular in one of their sites which is not essential for their replication, and in particular containing the nucleic acids coding for one of the following polypeptides :

* from amino acid (-20) to amino acid (187) represented in Fig. 2,
* from amino acid (1) to amino acid (187) represented in Fig. 2,
* from amino acid (1) to amino acid (187) represented in Fig. 2, preceded by Met.

The nucleic acids of the invention may be prepared either by a chemical method or by other methods.

A suitable method for preparing the nucleic acids (containing at most 200 nucleotides - or bp in the case of double-stranded nucleic acids) of the invention chemically comprises the following steps :

- DNA synthesis using the automatic β-cyanoethyl phosphoramidite method described in Bioorganic Chemistry 4; 274-325, 1986,
- cloning of the DNA thereby obtained into a suitable plasmid vector and recovery of the DNA by hybridization with a suitable probe.

A method for the chemical preparation of nucleic acids of length greater than 200 nucleotides - or bp (in the case of double-stranded nucleic acids) -comprises the following steps :

- assembling of chemically synthesized oligonucleotides, provided at their ends with different restriction sites, the sequences of which are compatible with the succession of amino acids in the natural peptide, according to the principle described in Proc. Nat. Acad. Sci. USA 80; 7461-7465, 1983,
- cloning of the DNA thereby obtained into a suitable plasmid vector and recovery of the desired nucleic acid by hybridization with a suitable probe.

Another method for preparing the nucleic acids of the invention from mRNA comprises the following steps:
- preparation of the cellular RNAs from the pituitary gland, according to the technique described by Ullrich and al., 1977, Science 196:1313-1319; and Chirgwin and al., 1979, Biochemistry 18, 5294-5299,
- recovery and purification of the mRNAs by passage of the total cellular RNAs employing chromatography with an immobilized oligo-(dT),
- synthesis of a strand of cDNA from the purified mRNAs according to the technique described in Gene 25:263, 1983,
- cloning of the nucleic acids thereby obtained in a suitable plasmid vector and recovery of the desired nucleic acid sequence using a suitable hybridization probe.

To prepare the nucleic acis of the invention, the following chemically synthetised oligonucleotide hybridization probes :
TAGCTCCACCTAATATTGATA
CAAACAGAGCCTGAACTGATG,
sequences of which have been derived from figure 2, may be employed under the hybridization conditions defined in Maniatis and al. (1982).

Synthesis of the cDNA strand and its subsequent in vitro amplification may also be realized using the Polymerase Chain Reaction method as described for example in Goblet and al., (1989), using two chemically synthetised amplimers defined from the sequence of figure 2. Two suitable amplimers are for example
ATGCCAGCCATGAACTCAGTC or CAGCAGATCACAGACAGCCAG and
CAGAGTGCAGTTTGCTTCTGG.

The amplified nucleic acid fragment may then be cloned as described previously.

Advantageous recombinant vectors of the invention contains :
- regulatory elements necessary to allow the expression of a sequence of amino acids of a polypeptide presenting growth promoting and/or osmoregulatory properties of tilapia or catfish in a cellular host and
- possibly a promoter, particularly an inducible promoter.

The invention also relates to cellular hosts which are transformed by recombinant vectors of the invention and comprising the regulation elements enabling the expression of the nucleic sequence coding for the polypeptide presenting growth promoting and/or osmoregulatory properties

of tilapia or catfish in this host.

An advantageous cellular host is E. coli transformed by anyone of the vectors of the invention.

The invention also relates to products for fish containing at least one of the polypeptides such as the ones of the invention, or mixtures thereof.

The invention also relates to food for fish containing, besides the classical nutrient ingredients at least anyone of the polypeptides of the invention, in an efficient amount such as 1μg to 1 mg/ of polypeptide/g of fish food per day.

## RESULTS

In the following disclosure one preferred protein product derived from the tilapia species Oreochromis niloticus will be described, by way of examplification only, namely one growth hormone (hereinafter referred to as TGH). The growth hormone product was obtained by isolating the genes coding for this hormone from a c-DNA library and expressing the cloned gene in strains of Escherichia coli, according to the procedures described hereafter.

### cDNA library construction

In order to facilitate the isolation of the hormone genes, a c-DNA library was constructed using m-RNA preparations isolated from the anterior part of the pituitary gland where these hormones are synthesized in large quantities. First was prepared total RNA from 1 gram of tissue using the guanidium isothiocyanate procedure (Ulrich et al., 1977; Chirgwin et al., 1979), whereafter the poly $A^+$ m -RNA fraction was purified by chromatography on oligo (dT) cellulose. The poly $A^+$ m-RNA was then used to synthesize double stranded cDNA according the procedure described by Gubler and Hoffman (1983). The double stranded cDNA was size fractionated by chromatography on sepharose 4B (Pharmacia) and the fractions containing DNA fragments larger than 700 base pairs were pooled. The blunt-ended c-DNA molecules were inserted into the pUC13 plasmid (Vieria and Messing, 1982) previously digested with Smal and dephosphorylated. The ligated products were then transformed into E. coli RR1XXM15 (lac Z, M15, F', lac I$^Q$, ZM15, proA) according the procedure of Mandel and Higa (1970), and clones containing recombinant plasmids were selected on Luria-broth plates containing 100 g/ml ampicillin, 0.5 mM isopropyl-$\beta$-D-thiogalactoside (IPTG) and 0,01 % 5-bromo-4-chloro-3-indolyl-$\beta$-D-galactopyranoside (X-gal). On these plates, transformants containing the religated pUC13 vector will yield blue colonies whereas the transformants containing recombinant pUC13 plasmids will give rise to white colonies. Starting from 2 g of polyA[+] m-RNA, we obtained a c-DNA library of approximatively 4.200 recombinant transformants.

Screening of the clones

2000 White colonies of the c-DNA library were transferred to nitrocellulose filters, grown overnight, lysed, neutralized and fixed as described in Maniatis (1980). The filters were then hybridized to appropriate $^{32}$p-labelled probes described below. Prehybridization and hybridization were carried out in 6X SSC-1X Denhardt's solution containing boiled, sheared salmon sperm DNA for 16 hours at a temperature of 45 C (1X SSC : 0.15 M Nacl / 0.015M Na citrate; 1X Denhardt's : 0,02% of each ficoll, polyvinylpyrolidone and bovine serum albumin). The filters were first washed in 6X SSC / 1X Denhardt's for 30 minutes at room temperature and then washed for 30 minutes at temperatures of 54 C.

The c-DNA clones coding for the tilapia growth hormone (TGH) were isolated by screening the c-DNA library with a $^{32}$P labelled probe isolated from the plasmid -containing the trout GH gene. As probe was used a Fokl-BGlll of 394 bp corresponding to the 5′ end of trout GH gene. The screening of 2000 recombinant white colonies of the cDNA library yielded 24 positive clones. The recombinant plasmids of each of these clones were extracted and sized by electrophoresis on 1 % agarose gel. Six of the plasmids were of similar size and contained a cDNA insert of 1000 bp corresponding to a full length TGH mRNA. Restriction analysis confirmed that all clones were identical, and one of these, designated ptiGH6, was chosen for further analysis. The entire cloned c-DNA fragment was transferred between the EcoRI-BamHI sites of the M13mp18 and M13mp19 vectors (Vieria and Messing, 1982), and its nucleotide sequence was determined using the dideoxynucleotide chain termination method of Sanger et al. (1977).

The nucleotide sequence of the TGH cDNA of ptiGH6 is presented in figure 2. The cDNA fragment has a length of 1000 nucleotides and contains an open reading frame of 621 nucleotide followed by a 3′ untranslated region carrying a polyadenylation signal located 18 nucleotides upstream the poly (A) tail. The polyA tail is about 150bp long. The ATG initiator codon at nucleotide 36 represents the putative translational start site as a part of a consensus initiation sequence PCCATGP (Kozak, 1986). The open reading frame encodes a protein of 204 amino acid residues with a calculated molecular weight of 23,114 daltons (Figure 2). The hydropathy profile of the deduced amino acid sequence, determined using the algorithm described by Kyte and Doolittle (1982), shows that the NH2 terminus is highly hydrophobic and exhibits all characteristics of a signal peptide. This peptide signal is supposedly cleaved off during processing to give a mature protein of 187 AA residues with 4 cysteine residues.

We have compared the derived amino acid sequence of the TGH protein with the amino acid sequences of other known fish growth hormones such as tuna, yellow tail, trout and salmon. We observed a similarity of 89 % between the TGH and tuna GH and of 84,5 % between TGH and yellow tail GH, while only 66 % similarity was found with trout GH and salmon GH. These results are in agreement with the taxonomic classification of these fish. Tilapia, tuna and yellow tail belong to the order of perciform, while trout and salmon belong to the order of salmoniform. Furthermore, these sequence comparisons further support above conclusion that the first 17 amino acis of the TGH protein comprise the signal sequence and that the mature protein starts at glutamine residue 1.

Expression of the TGH gene

The strategy used to express the TGH gene in E. coli was essentially as described by Studier and Moffat, 1986. In brief, the TGH gene are inserted behind the strong promoter of gene 10 of bacteriophage T7 in such a way that the translation of the inserted genes starts at the ribosome binding site of gene 10. Furthermore, we modified the beginning of the TGH gene so as to remove the signal peptide coding region, in such a way that the initiator ATG is followed immediately by the first codon of the mature TGH protein. In this way, the bacteria will synthesize directly the mature TGH protein without their respective signal peptides.

The general scheme for constructing the plasmids expressing the TPRL genes, illustrated in figure 4, involves two steps : in a first step we modified the 5′ ends of the TGH gene by combining an internal restriction enzyme fragment of the gene with a synthetic oligonucleotide containing an initiator ATG followed by the missing codons of the gene up to the restriction fragment. In a second step we inserted the modified 5′ ends of the gene together with the remaining parts of the gene behind the bacteriophage T7 promoter of the expression vector pARAE. The pARAE plasmid is derived from the plasmid vector pAR3040 (Rosenberg et al., 1987) which contains bacteriophage T7 promoter of gene 10 and its natural terminator for T7 RNA polymerase. In the pARAE derivative we have removed the PvuII-BglII and the EcoRI-EcoRV fragments and deleted the PstI site in the beta-lactamase gene.

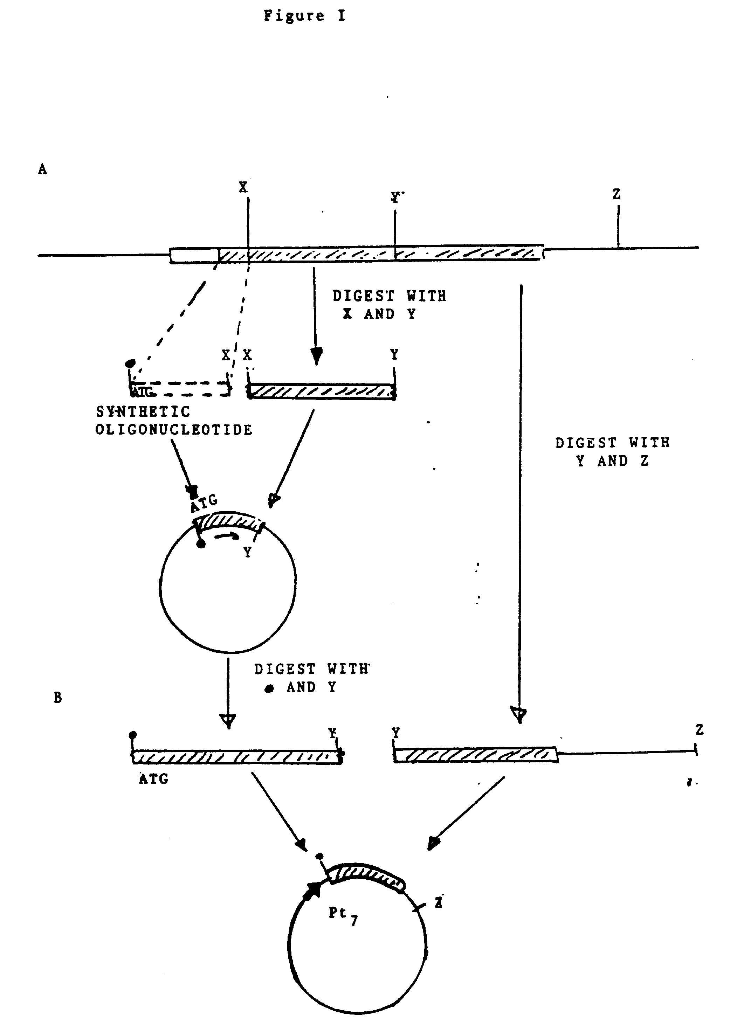

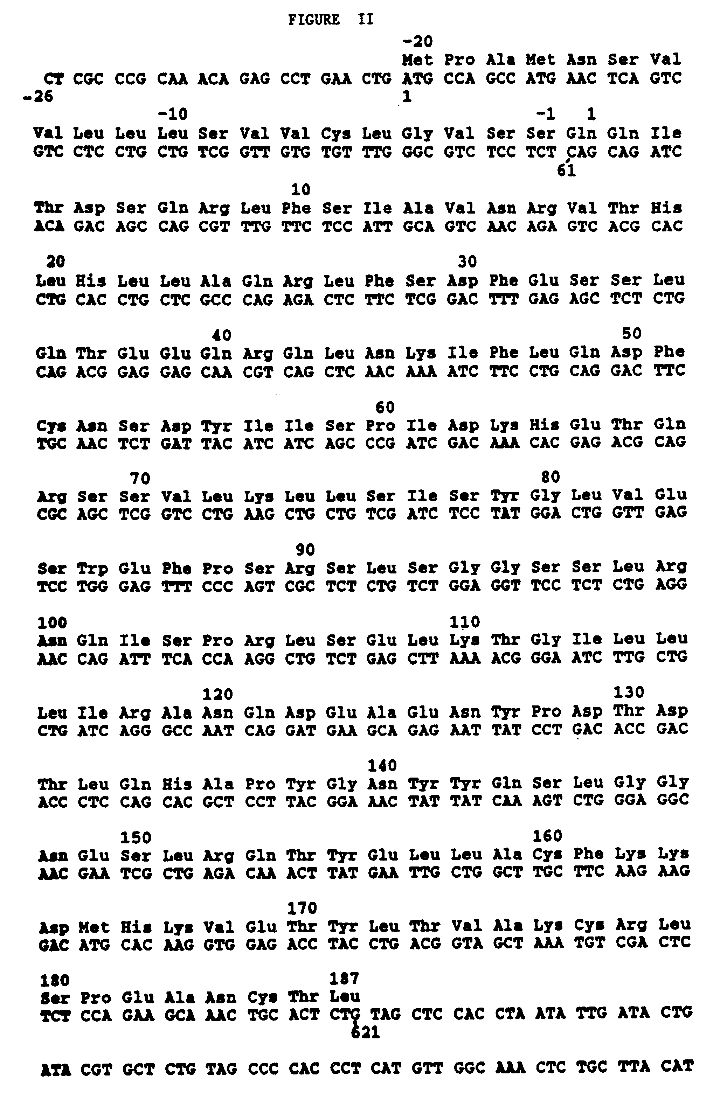

The same rationale was used for expressing the TGH gene (figure 1). In a first step we constructed the modified 5′ end of the TGH gene by combining a HindII-PvuI fragment of the gene starting at codon 15 with a synthetic oligonucleotide containing the initiator ATG followed by the first 14 codons of the mature TGH protein. In a second step we inserted the modified 5′ end of the TGH gene on a NdeI-PvuI fragment together with the remaining PvuI-BamHI fragment of the TGH gene in the expression vector pARAE.

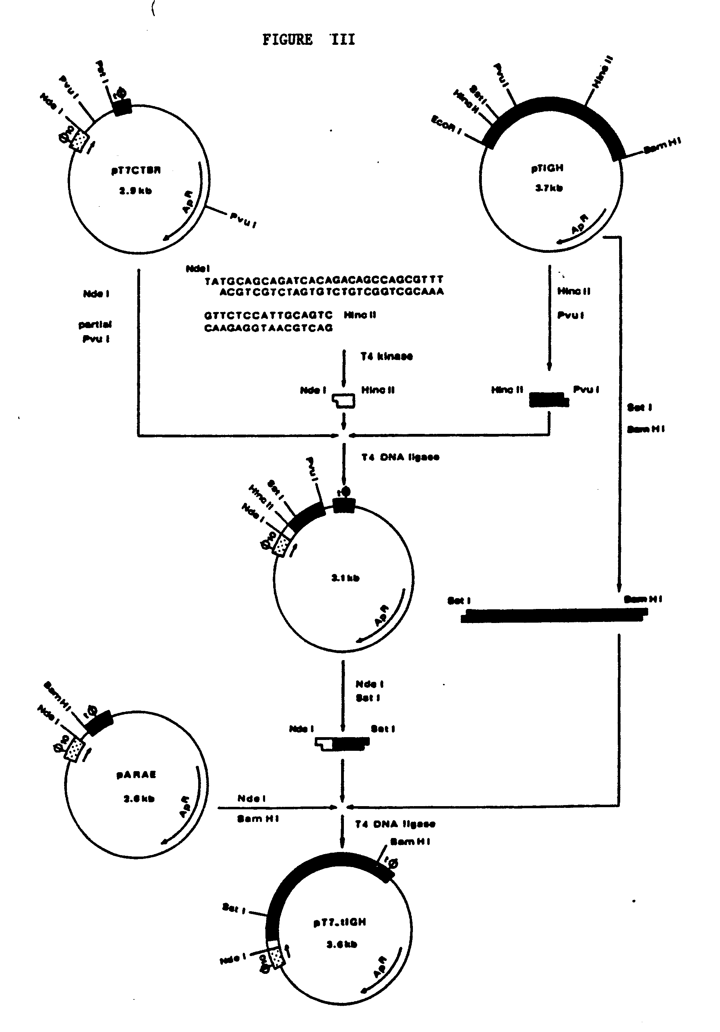

The construction of the plasmid pT7TGH diagrammed in figure 3 was performed as follows. In the first step we constructed the plasmid pTGHM containing the modified 5′ end of the TGH gene by inserting the synthetic oligonucleotide described in figure 3 together with HincII-PvuI fragment into the plasmid pT7CTBR. pT7CTBR is a derivative of pARAE and like pARAE it possesses a unique NdeI site. In addition, it also contains a PvuII and PstI site between the promoter and the terminator. pT7CTBR was cleaved with NdeI and partially with PvuI and the large fragment was ligated together with the synthetic oligonucleotide and the HincII-PvuI fragment isolated from the plasmid pTGH. The ligation mixture was transformed into *E. coli* HB101 and transformants containing the desired recombinant plasmid pTGHM were identified using standard plasmid analysis techniques. In a second step we constructed the plasmid pT7TGH by inserting 2 fragments of the TGH gene, respectively the NdeI-SStI fragment from plasmid pTGHM (containing the modified 5′ end of the tiGH gene) and the SstI-BamHi fragment from plasmid ptiGH (containing the rest of the TGH cDNA) into the expression vector pARAE. The two fragments were purified by agarose gel electrophoresis after digesting the two plasmids with the respective restriction enzymes and ligated together with the plasmid pARAE digested with the enzymes NdeI and BamHi. The ligation mixture was then used to transform *E. coli* HB101 and transformants containing the desired recombinant plasmid, designated pT7TGH, were identified using standard plasmid analysis techniques.

In order to express the TGH protein, the pT7TGH plasmid was introduced into the *E. coli* strain BL21 (DE3) described earlier. *E. coli* BL21-(DE3) containing the pT7TGH plasmid were grown overnight in 5ml Luria-broth in the presence of 100 μg/ml ampicillin. Of this culture, 0.4 ml were used to inoculate 10 ml of the same medium supplemented with ampicillin (100 μg/ml). and 0.4 mM IPTG was added when the culture reached A₆₀₀m = 0.8. After three hours, the cells were harvested and lysed in SDS sample buffer (Laemmli, 1970) and analyzed by sodium dodecylsulfate polyacrylamide gel electrophoresis (SDS-PAGE) with Coomassie blue staining. Comparison of the total cell proteins in extracts of non-induced cultures and IPTG induced cultures shows that a new abundant 24 kd protein is produced.

The newly synthesized TGH protein is made in large amounts, reaching 25 % of the total cellular protein. Western blot analysis of the SDS PAGE using a salmon growth hormone antiserum shows that the newly synthesized proteins cross reacts with the antiserum, thus demonstrating that the TGH protein corresponds to the tilapia growth hormone.

The overproduced TGH protein is found to be contained within inclusion bodies, which are readily purified after lysing the cells by sonication and centrifugation. The growth hormone protein can be easily purified by solubilizing the purified inclusion bodies in the presence of 8 M urea, whereafter the solution is dialyzed against buffers containing protease inhibitors. The protein is further purified by chromatography on Sephadex G100.

## FIGURE LEGENDS

### Figure 1 :

General scheme for the expression of mature TGH proteins in E. Coli.

(a) Scheme for modifying the 5′ end of the gene so as to delete the signal peptide coding region (open box).

(b) Scheme for engineering modified genes into the bacteriophage T7 promoter (PT7) vectors.

### Figure 2 :

Nucleotide sequence of the TGH cDNA fragment of plasmid pTGH and amino acid sequence of the TGH protein derived from the nucleotide sequence. The amino acids of the mature TGH are numbered and the negatively numbered amino acids correspond to the signal peptide.

The total nucleotide sequence is numbered from (-26) to (z).

The coding sequence is numbered from (1) to (621).

### Figure 3 :

Construction of the PT7TGH expression vector.

(a) Construction of the modified 5' end of the TGH gene.

(b) Construction of the modified TGH gene behind the bacteriophage T7 promoter.

## REFERENCES

- Agellon L.B. and Chen T.T., (1986). Rainbow trout growth hormone : molecular cloning of cDNA and expression in Escherichia coli. DNA 5, 463-471.

- Chirgwin J.M., Prsybyla A.E., MacDonald R.J. and Rutter W.J., (1979) Isolation of biologically active ribonucleic acid from sources enriched in ribonuclease. Biochemistry 18, 5294-5299.

- Gonzales-Villasenor L.I., Shang P., Chen T.T. and Powers D.A., (1988). Molecular cloning and sequencing of coho salmon growth hormone cDNA. Gene 65, 239-246.

- Gubler U. and Hoffman D.J., (1983). A simple and very efficient method for generating cDNA libraries. Gene 25, 263-269.

-Kozak M., (1986). Point mutations define a sequence flanking the ADG initiator codon that modulates translating by eukaryotic ribosomes. Cell 44, 283-292.

- Kyte J. and Doolittle R.F., (1982). A simple method for displaying the hydrophatic character of a protein. J. Mol. Biol. 157, 105-132.

- Laemmli U.K., (1970). Cleavage of structural proteins during the assembly of the head of bacteriophage T4. Nature (London), 227, 680-685.

- Mandel M. and Niga A., (1970). Calcium dependent bacteriophage DNA infection. J. Mol. Biol. 53, 159-162.

- Maniatis T., Fritsch E.F. and Sambrook J.. J. "Molecular Cloning", Cold Spring Harbor Laboratory, 1982.

- Nicoll C.S., Steiny S.S., Kind D., Nishioka R.S., Mayer G.L., Eberhardt N.L., Baxter J.D., Yamanaka M.K., Miller J.A., Seilhamer J.J., Schilling J.W. and Johnson L.K. (1987). The primary structure of coho salmon growth hormone and its cDNA. Gen. Comp. Endocrinol. 68, 387-399.

- Rosenberg A.H., Lade B.W., Chin D., Lin S., Dunn J.J. and Studier F.W., (1987). Vectors for selective expression of cloned cDNAs by T7 RNA polymerase. Gene 56, 125-135.

- Sanger F., Nickler S. and Coulson A.R., (1977). DNA sequencing with chain-terminating inhibitors. Proc. Natl. Acad. Sci. USA 74, 5463-5467.

- Sato N., Watanabe K, Murata K., Sabaguchi M., Kariya Y., Kimura S., Nonaka M. and Kimura A., (1988). Molecular cloning and nucleotide sequence of tuna growth hormone cDNA. Biochem. Biophys. Acta 949, 35-42.

- Sekine S., Mizukami T., Nishi T., Kuwana Y., Saito A., Sato M., Itoh S. and Kawauchi H., (1985). Cloning and expression of cDNA for salmon growth hormone in Escherichia coli. Proc. Natl. Acad. Sci. USA 82, 4306-4310.

- Studier F.W. and Moffat B.A., (1986). Use of bacteriophage T7 RNA polymerase to direct selective high-level expression of cloned genes. J. Mol. Biol. 186,

- Prunet and Bornancin, (1988) 113-130.

- Ullrich A., Shine J., Chirgwin J., Pictet R., Tischer E., Rutter W.J. and Goodman H.M., (1977). Rat insulin genes : construction of plasmids containing the coding sequences. Science 196 : 1313-1319.

- Viera J. and Messing J., (1982). The pUC plasmids, an M13mp7 derived system for insertion mutagenesis and sequencing with synthetic universal primers. Gene 19, 259-268.

- Yanish-Perron C., Vieira J. and Messing J., (1985). Improved M13 phage cloning vectors and host strains : nucleotide sequences of the M13mp18 and pUC19 vectors. Gene 33, 103-119.

- Goblet C., Prost E. and Whalen R.G., Nucleic Acid Research, 17, 2144, 1989, "One step amplification of transcripts in total RNA using Polymerase Chain Reaction".

## Claims

1. Recombinant stable polypeptide comprising in its polypeptide chain an aminoacid sequence contained in one of the following amino sequences :

* from amino acid (-20) to amino acid (187) represented in Fig. 2,

* from amino acid (1) to amino acid (187) represented in Fig. 2,

* from amino acid (1) to amino acid (187) represented in Fig. 2, preceded by Met.

2. Recombinant polypeptide according to claim 1, containing in its polypeptide chain the following amino acid sequence :

* from amino acid (-20) to amino acid (187) represented in Fig. 2,

* from amino acid (1) to amino acid (187) represented in Fig. 2,

* from amino acid (1) to amino acid (187) represented in Fig. 2, preceded by Met,

- or constituted by one of the abovesaid amino acid sequences.

3. Process for preparing a stable recombinant polypeptide according to claim 1 or 2, which comprises the following steps :

- a cellular host, which is previously transformed by an appropriate vector containing a nucleic acid coding for polypeptides having

. the polypeptide sequence of the natural growth hormones of tilapia or catfish or

. a polypeptide sequence containing part or the totality of the sequence of the natural growth hormones of tilapia or catfish, in so far as the polypeptide sequence produced has growth properties, is cultivated in an appropriate medium at a temperature from about 28°C to aobut 42°C depending on the selected microorganism and

- the polypeptide produced by said transformed cellular host is recovered from the above said medium culture and, if need be, purified.

4. Process according to claim 3 wherein the nucleic acids coding for the natural polypeptides presenting growth promoting and/or osmoregulatory properties are under the control of regulatory elements, such as a promoter recognized by the RNA polymerase of the host organism, a translation initiation and terminatory signal.

5. Process according to anyone of claims 3 or 4, wherein the cellular host is E. coli, the growth of which is stopped at the exponential phase and the polypeptide produced by said transformed cellular host is recovered from the medium culture free from other proteins and exocellular enzymes liable to be secreted by E. coli.

6. Process according to anyone of claims 3 or 4, wherein the cellular host is yeast.

7. Process according to anyone of claims 3 to 6, wherein the recombinant polypeptide is preceded by a signal peptide, particularly by a signal peptide having the same amino acid sequence as the natural signal peptide in tilapia or catfish.

8. Process according to anyone of claims 3 to 6, wherein the recombinant polypeptide is preceded by a methionine.

9. Nucleic acid comprising a nucleotidic chain coding for the polypeptides according to claims 1 or 2.

10. Nucleic acid comprising a part or the totality of one of the following nucleotidic sequences: * from nucleotide (-26) to nucleotide (z) of Fig. 2,

* from nucleotide (1) to nucleotide (621) of figure 2,

* from nucleotide (61) to nucleotide (621) of Fig. 2,

* from nucleotide (61) to nucleotide (621) of Fig. 2, preceded by the AUG codon,

or is constituted by one of the above mentioned nucleotidic sequences.

11. Recombinant vector, particularly for the cloning and/or expression, particularly of the plasmid, cosmid or phage type, containing a nucleic acid coding for a natural polypeptide - presenting growth promoting and/or osmoregulatory properties - of tilapia or catfish in particular in one of its site which is not essential for its replication, and in particular containing the nucleic acids coding for one of the following polypeptides :

* from amino acid (-20) to amino acid (187) represented in Fig. 2,

* from amino acid (1) to amino acid (187) represented in Fig. 2,

* from amino acid (1) to amino acid (187) represented in Fig. 2, preceded by Met,

12. Recombinant vector according to claim 11 containing

- regulatory elements necessary to allow the expression of a sequence of amino acids of a polypeptide presenting growth promoting and/or osmoregulatory properties of tilapia or catfish in a cellular host and

- possibly a promoter, particularly an inducible promoter.

13. Cellular host which is transformed by a recombinant vector according to any one of claim 11 and 12 and comprising the regulation elements enabling the expression of the nucleic sequence coding for the polypeptide presenting growth promoting and/or osmoregulatory properties - of tilapia or catfish in this host.

14. Cellular host according to claim 16 which is E. Coli transformed by a the vector according to claim 11 or 12.

15. Expression product of a nucleic acid expressed by a transformed cellular host according to claim 13 or 14.

16. Composition for fish containing at least one of the polypeptides of claims 1 and 2, or mixtures thereof in an efficient amount such as 5 ng to 1 $\mu$g of polypeptide / g of fish per day, when administered by injection.

17. Food for fish, containing besides the classical nutrient ingredients, at least anyone of the polypeptides such as the ones obtained according to anyone of claims 1 and 2, in an efficient amount such as 1 $\mu$g to 1 mg of polypeptide / g of fish food per day.

Claims for the following Contracting State: Es

1. Process for preparing a recombinant stable polypeptide comprising in its polypeptide chain an aminoacid sequence contained in one of the following amino sequences :

* from amino acid (-20) to amino acid (187) represented in Fig. 2,

* from amino acid (1) to amino acid (187) represented in Fig. 2,

* from amino acid (1) to amino acid (187) represented in Fig. 2, preceded by Met, said process comprising the following steps:

- a cellular host, which is previously transformed by an appropriate vector containing a nucleic acid coding for polypeptides having

. the polypeptide sequence of the natural growth hormones of tilapia or catfish or

. a polypeptide sequence containing part or the totality of the sequence of the natural growth hormones of tilapia or catfish, in so far as the polypeptide sequence produced has growth properties,
is cultivated in an appropriate medium at a temperature from about 28°C to about 42°C depending on the selected microorganism and
- the polypeptide produced by said transformed cellular host is recovered from the above said medium culture and, if need be, purified.

2. Process for preparing a recombinant polypeptide according to claim 1, containing in its polypeptide chain the following amino acid sequence :
* from amino acid (-20) to amino acid (187) represented in Fig. 2,
* from amino acid (1) to amino acid (187) represented in Fig. 2,
* from amino acid (1) to amino acid (187) represented in Fig. 2, preceded by Met,
- or constituted by one of the abovesaid amino acid sequences.

3. Process according to claim 1 or 2 wherein the nucleic acids coding for the natural polypeptides presenting growth promoting and/or osmoregulatory properties are under the control of regulatory elements, such as a promoter recognized by the RNA polymerase of the host organism, a translation initiation and terminatory signal.

4. Process according to anyone of claims 1 to 3, wherein the cellular host is E. coli, the growth of which is stopped at the exponential phase and the polypeptide produced by said transformed cellular host is recovered from the medium culture free from other proteins and exocellular enzymes liable to be secreted by E. coli.

5. Process according to anyone of claims 1 to 3, wherein the cellular host is yeast.

6. Process according to anyone of claims 1 to 5, wherein the recombinant polypeptide is preceded by a signal peptide, particularly by a signal peptide having the same amino acid sequence as the natural signal peptide in tilapia or catfish.

7. Process according to anyone of claims 1 to 5, wherein the recombinant polypeptide is preceded by a methionine.

8. Process for preparing a recombiannt stable polypeptide comprising in its polypeptide chain an aminoacid sequence contained in one of the following amino sequences :
* from amino acid (-20) to amino acid (187) represented in Fig. 2,
* from amino acid (1) to amino acid (187) represented in Fig. 2,
* from amino acid (1) to amino acid (187) represented in Fig. 2, preceded by Met, said process being characterized by the fact that, preferably starting from the C-terminal amino acid, the successive aminoacyls in the requisite order, or aminoacyls and fragments formed beforehand and already containing several aminoacyl residues in the appropriate order, or alternatively several fragments prepared in this manner beforehand, are condensed successively in pairs, it being understood that care will have been taken to protect beforehand all the reactive groups carried by these aminoacyls or fragments except for the amine groups of one and carboxy group of the other, or vice versa, which must normally participate in peptide bond formation, in particular after activation of the carboxyl group, according to methods known in peptide synthesis, and so on, proceeding stepwise up to the N-terminal amino acid.

9. Process for preparing a nucleic acid comprising a nucleotidic chain coding for the polypeptides according to claims 1 or 2, particularly a nucleic acid comprising a part or the totality of one of the following nucleotidic sequences:
* from nucleotide (-26) to nucleotide (z) of Fig. 2,
* from nucleotide (1) to nucleotide (621) of figure 2,
* from nucleotide (61) to nucleotide (621) of Fig. 2,
* from nucleotide (61) to nucleotide (621) of Fig. 2, preceded by the AUG codon,
or is constituted by one of the above mentioned nucleotidic sequences,
comprising the following steps :
- when the nucleic acid to be prepared comprises at most 200 nucleotides :
. DNA synthesis using the automatic $\beta$-cyanoethyl phosphoramidite method described in Bioorganic Chemistry 4; 274-325, 1986,
. cloning of the DNA thereby obtained into a suitable plasmid vector and recovery of the DNA by hybridization with a suitable probe,
- and when the nucleic acid to be prepared comprises more than 200 nucleotides :
. assembling of chemically synthesized oligonucleotides, provided at their ends with different restriction sites, the sequences of which are compatible with the succession of amino acids in the natural peptide, according to the principle described in Proc. Nat. Acad. Sci. USA 80; 7461-7465, 1983,
. cloning of the DNA thereby obtained into a suitable plasmid vector and recovery of the desired nucleic acid by hybridization with a suitable probe.

10. Process for preparing from mRNA a nucleic acid comprising a nucleotidic chain coding for the polypeptides according to claims 1 or 2, particularly a nucleic acid comprising a part or the totality of one of the following nucleotidic sequences:
* from nucleotide (-26) to nucleotide (z) of Fig. 2,
* from nucleotide (1) to nucleotide (621) of figure 2,
* from nucleotide (61) to nucleotide (621) of Fig. 2,
* from nucleotide (61) to nucleotide (621) of Fig. 2, preceded by the AUG codon,
or is constituted by one of the above mentioned nucleotidic sequences,

said process comprising the following steps :

- preparation of the cellular RNAs from the pituitary gland, according to the technique described by Ullrich and al., 1977, Science 196:1313-1319; and Chirgwin and al., 1979, Biochemistry 18, 5294-5299,

- recovery and purification of the mRNAs by passage of the total cellular RNAs employing chromatography wit an immobilized oligo-(dT),

- synthesis of a strand of cDNA from the purified mRNAs according to the technique described in Gene 25:263, 1983,

- cloning of the nucleic acids thereby obtained in a suitable plasmid vector and recovery of the desired nucleic acid sequence using a suitable hybridization probe.

11. Process for preparing a nucleotide probe of the following sequence :

TAGCTCCACCTAATATTGATA

CAAACAGAGCCTGAACTGATG,

characterized by the fact that DNA synthesis is carried out by using the automatic $\beta$-cyanoethyl phosphoramidite method described in Bioorganic Chemistry 4; 274-325, 1986.

12. Process for detecting a nucleic acid sequence defined in claims 9 and 10 characterized by the fact that a probe according to claim 11 is used under the hybridization conditions defined in Maniatis and al., "Molecular Cloning", Cold Spring Harbor Laboratory, 1982.

13. Food for fish, containing besides the classical nutrient ingredients, at least anyone of the polypeptides such as the ones obtained according to anyone of claims 1 to 8, in an efficient amount such as 1 $\mu$g to 1 mg of polypeptide / g of fish food per day.

Figure I

FIGURE II

```
                                               -20
                            Met Pro Ala Met Asn Ser Val
   CT CGC CCG CAA ACA GAG CCT GAA CTG ATG CCA GCC ATG AAC TCA GTC
-26                                            1

            -10                                        -1    1
Val Leu Leu Leu Ser Val Val Cys Leu Gly Val Ser Ser Gln Gln Ile
GTC CTC CTG CTG TCG GTT GTG TGT TTG GGC GTC TCC TCT CAG CAG ATC
                                                          61

                             10
Thr Asp Ser Gln Arg Leu Phe Ser Ile Ala Val Asn Arg Val Thr His
ACA GAC AGC CAG CGT TTG TTC TCC ATT GCA GTC AAC AGA GTC ACG CAC

 20                                          30
Leu His Leu Leu Ala Gln Arg Leu Phe Ser Asp Phe Glu Ser Ser Leu
CTG CAC CTG CTC GCC CAG AGA CTC TTC TCG GAC TTT GAG AGC TCT CTG

                      40                                      50
Gln Thr Glu Glu Gln Arg Gln Leu Asn Lys Ile Phe Leu Gln Asp Phe
CAG ACG GAG GAG CAA CGT CAG CTC AAC AAA ATC TTC CTG CAG GAC TTC

                                         60
Cys Asn Ser Asp Tyr Ile Ile Ser Pro Ile Asp Lys His Glu Thr Gln
TGC AAC TCT GAT TAC ATC ATC AGC CCG ATC GAC AAA CAC GAG ACG CAG

            70                                      80
Arg Ser Ser Val Leu Lys Leu Leu Ser Ile Ser Tyr Gly Leu Val Glu
CGC AGC TCG GTC CTG AAG CTG CTG TCG ATC TCC TAT GGA CTG GTT GAG

                             90
Ser Trp Glu Phe Pro Ser Arg Ser Leu Ser Gly Gly Ser Ser Leu Arg
TCC TGG GAG TTT CCC AGT CGC TCT CTG TCT GGA GGT TCC TCT CTG AGG

100                                        110
Asn Gln Ile Ser Pro Arg Leu Ser Glu Leu Lys Thr Gly Ile Leu Leu
AAC CAG ATT TCA CCA AGG CTG TCT GAG CTT AAA ACG GGA ATC TTG CTG

                      120                               130
Leu Ile Arg Ala Asn Gln Asp Glu Ala Glu Asn Tyr Pro Asp Thr Asp
CTG ATC AGG GCC AAT CAG GAT GAA GCA GAG AAT TAT CCT GAC ACC GAC

                            140
Thr Leu Gln His Ala Pro Tyr Gly Asn Tyr Tyr Gln Ser Leu Gly Gly
ACC CTC CAG CAC GCT CCT TAC GGA AAC TAT TAT CAA AGT CTG GGA GGC

            150                                     160
Asn Glu Ser Leu Arg Gln Thr Tyr Glu Leu Leu Ala Cys Phe Lys Lys
AAC GAA TCG CTG AGA CAA ACT TAT GAA TTG CTG GCT TGC TTC AAG AAG

                            170
Asp Met His Lys Val Glu Thr Tyr Leu Thr Val Ala Lys Cys Arg Leu
GAC ATG CAC AAG GTG GAG ACC TAC CTG ACG GTA GCT AAA TGT CGA CTC

180                               187
Ser Pro Glu Ala Asn Cys Thr Leu
TCT CCA GAA GCA AAC TGC ACT CTG TAG CTC CAC CTA ATA TTG ATA CTG
                                621


ATA CGT GCT CTG TAG CCC CAC CCT CAT GTT GGC AAA CTC TGC TTA CAT
```

## FIGURE II (suite)

GTG TTA GCA TTA GCA ATA GGA TAA TAA TAG CAG TGG TAA TCG TGA CAT

CAG AAC GTT TTT TCT GAC ATA ACT GTG ATG CAA GGT GTG AAC GGG AAT

AAT GTT ATC TGT GAA ATA AAT GTG TTG CAT TGA AAA AAA AAA AAA AAA

AAA AAA AAA AAA AAA AAA AAA AAA

z

# FIGURE III

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | DNA, vol. 8, no. 4, 1989, pages 271-278; F. RENTIER-DELRUE et al.: "Tilapia growth hormone: molecular cloning of cDNA and expression in Escherichia coli" * Whole document * | 1-5,7,9 -17 | C 12 N 15/18<br>C 12 P 21/02<br>C 07 K 13/00<br>C 12 N 1/21<br>A 23 K 1/165<br>A 61 K 37/36 //<br>(C 12 N 1/21<br>C 12 R 1:19 ) |
| X | CHEMICAL ABSTRACTS, vol. 87, no. 19, 7th November 1977, page 334, abstract no. 149071c, Columbus, Ohio, US; W.C. CLARKE et al.: "Effect of teleost pituitary growth hormone on growth of Tilapia mossambica and on growth and seawater adaptation of sockeye salmon (Oncorhynchus nerka)", & GEN. COMP. ENDOCRINOL. 1977, 33(2), 174-8 * Abstract * | 1,2,15- 17 | |
| Y | IDEM | 3-14 | |
| X | PROC. NATL. ACAD. SCI. USA; vol. 82, November 1985, pages 7490-7494, US; J.L. SPECKER et al.: "Isolation and partial characterization of a pair of prolactins released in vitro by the pituitary of a cichlid fish, Oreochromis mossambicus" * Page 7490, column 1, last paragraph - page 7492, figure 2 * | 1,2,15 | TECHNICAL FIELDS SEARCHED (Int. Cl.5)<br><br>C 12 N<br>C 12 P<br>C 07 K<br>A 61 K<br>A 23 K |
| Y | IDEM | 3-14,16 ,17 | |
| | --- | -/- | |

The present search report has been drawn up for all claims

| Place of search | Date of completion of the search | Examiner |
|---|---|---|
| THE HAGUE | 15-03-1990 | DESCAMPS J.A. |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | BIOCHEM. BIOPHYS. ACTA, vol. 949, 1988, pages 35-42, Elsevier Science Publishers B.V.; N. SATO et al.: "Molecular cloning and nucleotide sequence of tuna growth hormone cDNA" * Whole article * | 3-4,16, 17 | |
| Y | EP-A-0 239 075 (KYOWA HAKKO KOGYO CO., LTD) * Whole document * | 3-14,16 ,17 | |
| Y | EP-A-1 664 44 (KYOWA HAKKO KOGYO CO., LTD) * Whole document * | 3-14,16 ,17 | |
| Y | EP-A-0 213 357 (KYOWA HAKKO KOGYO K.K.) * Whole document * | 16,17 | |

TECHNICAL FIELDS SEARCHED (Int. Cl.5)

The present search report has been drawn up for all claims

| Place of search | Date of completion of the search | Examiner |
|---|---|---|
| THE HAGUE | 15-03-1990 | DESCAMPS J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)